# EUROPEAN PATENT SPECIFICATION

(11) **EP 3 699 209 B1**
(45) Date of publication and mention of the grant of the patent: **27.10.2021**
(21) Application number: 18867569.8
(22) Date of filing: 20.09.2018
(51) Int. Cl.: A61L 27/34, A61L 27/50, A61L 29/08, A61L 29/14, A61L 31/10, A61L 31/14, C09D 4/00, A61L 33/06, C09D 135/00, C09D 133/12, C08F 222/38, C08F 222/10

(54) **CURABLE COMPOSITION, FILM, CURED PRODUCT, AND MEDICAL MEMBER**
HÄRTBARE ZUSAMMENSETZUNG, FILM, GEHÄRTETES PRODUKT UND MEDIZINISCHES ELEMENT
COMPOSITION DURCISSABLE, FILM, OBJET DURCI, ET ÉLÉMENT À USAGE MÉDICAL

(30) Priority: 18.10.2017 JP 2017201902
(43) Date of publication of application: 26.08.2020
(73) Proprietor: FUJIFILM Corporation, Tokyo 106-8620 (JP)
(72) Inventor: YAMAMOTO, Yosuke, Minamiashigara-shi Kanagawa 250-0193 (JP); AMAO, Akihito, Minamiashigara-shi Kanagawa 250-0193 (JP)
(74) Representative: Hoffmann Eitle
(86) International application number: PCT/JP2018/034731
(87) International publication number: WO 2019/077929

(56) References cited:
- EP-A1- 3 064 192
- WO-A1-2016/067795
- WO-A1-2017/018146
- WO-A1-2017/018146
- WO-A1-2017/073437
- WO-A1-2018/076068
- JP-A- S50 153 047
- JP-A- S62 232 410
- JP-A- 2013 053 082
- JP-A- 2017 105 716
- JP-A- 2017 105 716
- JP-A- 2018 022 023
- US-A1- 2014 120 326

## Description

### BACKGROUND OF THE INVENTION

### 1. Field of the Invention

The present invention relates to a curable composition, a film, a cured product, and a medical member.

### 2. Description of the Related Art

Originally, it is desired that artificial organs, medical instruments, and the like are made of a material which is compatible with a substance constituting a living body and is hardly contaminated. For example, it is desired that artificial blood vessels, catheters, stents, artificial bones, and dentures that are introduced into a human body for a long period of time do not cause an inflammatory response and rejection. In addition, the replacement of those artificial organs or medical instruments caused by contamination imposes a burden on the patient. Therefore, it is desired that artificial organs, medical instruments, and the like are formed of a material which hardly interacts with biological materials such as proteins, blood cells, and cells. That is, it is desired that artificial organs, medical instruments, and the like are formed of a material difficult for the aforementioned biological materials to be attached (adhere).

For example, WO2016/067795A discloses "a material nonadhesive to biological materials containing a polymer compound (A) containing a repeating unit derived from a sulfobetaine monomer having a predetermined structure". Examples in WO2016/067795A specifically disclose a curable composition containing a sulfobetaine monomer having the aforementioned predetermined structure and a (meth)acrylate-based monomer or N-[tris(3-acrylamidopropoxymethyl)methyl] acrylamide as a crosslinking agent and a cured product of the curable composition (material nonadhesive to biological materials).

JP2017 105716 A discloses self-adhesive dental composite resin for treating dental caries, comprising multifunctional (meth)acrylamide compounds. Such compositions exhibit adhesiveness with respect to dentine.

EP 3 064 192 A1 discloses dental materials via light-curing with enhanced mechanical properties based on monomers being polyfunctional compounds.

### SUMMARY OF THE INVENTION

The cured product (material nonadhesive to biological materials) is also required to have excellent substrate adhesion. In the living body, artificial organs, medical instruments, and the like are generally used in a situation where these contact body fluids (for example, saliva, blood, and the like), which contain water as a main component, with high frequency. Therefore, "have excellent substrate adhesion" mentioned herein means that even after a substrate with a film, which includes a substrate and a film formed on the substrate, is immersed in an aqueous liquid, the film is not peeled or hardly peeled from the substrate. Examples of the substrate include medical instruments such as a denture and an artificial bone.

The inventors of the present invention prepared the curable composition described in Examples of WO2016/067795A and examined the physical properties of the cured product of the composition. As a result, they have found that the substrate adhesion and the biocompatibility (properties of inhibiting the adhesion of biological materials) have a trade-off relationship. That is, it has been revealed that in order for both the substrate adhesion and biocompatibility to satisfy the currently required level, the cured product needs to be further improved.

Therefore, an object of the present invention is to provide a curable composition capable of providing a cured product excellent in both the substrate adhesion and biocompatibility.

Another object of the present invention is to provide a cured product and a film that are excellent in both the substrate adhesion and biocompatibility.

Another object of the present invention is to provide a medical member comprising the cured product.

In order to achieve the above objects, the inventors of the present invention carried out intensive examinations. As a result, the inventors have found that in a case where the curable composition contains a compound, which has a predetermined structure containing a substituted or unsubstituted acrylamide group and a substituted or unsubstituted acrylate group, and a compound, which has a predetermined structure containing a (meth)acrylamide group, or a betaine monomer, the above objects can be achieved. Based on the finding, the inventors have accomplished the present invention.

According to the present invention, it is possible to provide a curable composition capable of providing a cured product which is excellent in both the substrate adhesion and biocompatibility.

Furthermore, according to the present invention, it is possible to provide a cured product and a film which are excellent in both the substrate adhesion and biocompatibility.

In addition, according to the present invention, it is possible to provide a medical member comprising the cured product.

### DESCRIPTION OF THE PREFERRED EMBODIMENTS

Hereinafter, the present invention will be specifically described.

The following constituents will be described based on the typical embodiments of the present invention in some cases, but the present invention is not limited to the embodiments.

In the present specification, a range of numerical values described using "to" means a range including numerical values listed before and after "to" as a lower limit and an upper limit.

In the present specification, "(meth)acrylamide" has a concept including either or both of the acrylamide and methacrylamide. The same is true of the terms such as "(meth)acryl", "(meth)acrylate", and "(meth)acryloyl".

In the present specification, in a case where there is a plurality of substituents, linking groups, and the like (hereinafter, described as substituents and the like) marked with specific reference signs, or in a case where a plurality of substituents and the like are simultaneously specified, the substituents and the like may be the same as or different from each other. The same is true of a case where the number of substituents and the like is specified.

Furthermore, in the present specification, in a case where there is no description regarding whether or not a group (atomic group) is substituted or unsubstituted, the group includes both the group having no substituent and group having a substituent. For example, "alkyl group" includes not only an alkyl group having no substituent (unsubstituted alkyl group) but also an alkyl group having a substituent (substituted alkyl group).

In the present specification, "biological material" is a term that means a wide variety of materials including materials constituting the living body and materials involved in the living body. For example, the term means materials including proteins, cells, tissues as groups of cells, peptides, vitamins, hormones, blood cells, antigens, antibodies, bacteria, viruses, and the like.

Furthermore, in the present specification, the term "properties of inhibiting the adhesion of biological materials" means the properties of perfectly preventing adhesion and also suppressing adhesion (reducing adhesion) before and after application even though adhesion occurs. Therefore, the term has a concept including not only the prevention of adhesion but also the inhibition of adhesion.

### [Curable composition]

The curable composition according to an embodiment of the present invention (hereinafter, referred to as "the composition according to the embodiment of the present invention" as well) contains one or more kinds of polyfunctional compounds (hereinafter, referred to as "specific polyfunctional compound" as well) selected from the group consisting of a compound represented by Formula (1) which will be described later, a compound represented by Formula (2) which will be described later, and a compound represented by Formula (3) which will be described later, and one or more kinds of compounds selected from the group consisting of a compound represented by formula (A) which will be described later (hereinafter, referred to as "specific polyfunctional (meth)acrylamide compound" as well) and a betaine monomer.

One of the characteristics of the composition according to the embodiment of the present invention is, for example, that the specific polyfunctional compounds and the specific polyfunctional (meth)acrylamide compound or the betaine monomer having excellent hydrophilicity are used in combination.

The inventors of the present invention have found a cured product having excellent biocompatibility tends to exhibit high hydrophilicity, but exhibits poor adhesion to a substrate in an aqueous liquid. In other words, the inventors have found that in a case where a cured product is formed on a substrate by using only the components such as the specific polyfunctional (meth)acrylamide compound and the betaine monomer, although the cured product itself has excellent biocompatibility, in a case where the substrate is hydrophobic and is immersed in an aqueous liquid, the cured product is easily peeled from the substrate.

Regarding the above finding, the inventors have revealed that the cured product, which is obtained from the composition containing the specific polyfunctional (meth)acrylamide compound or the betaine monomer having excellent hydrophilicity, and the specific polyfunctional compound is excellent in both the substrate adhesion and biocompatibility.

Although the reason is unclear, it is considered that the above characteristics may result mainly from the structure of the specific polyfunctional compound. The specific polyfunctional compound has at least one or more substituted or unsubstituted acrylamide groups exhibiting hydrophilicity and at least one or more substituted or unsubstituted acrylate groups exhibiting hydrophobicity, and has a predetermined structure in which the total number of the acrylamide group and the acrylate group is 3 or greater. In the cured product obtained from the composition according to the embodiment of the present invention, the repeating unit derived from the specific polyfunctional compound acts as a linker, and play a role of linking the repeating unit derived from the specific polyfunctional (meth)acrylamide compound or the repeating unit derived from the betaine monomer. In addition, it is considered that because the specific polyfunctional compound itself contains a substituted or unsubstituted acrylamide group exhibiting hydrophilicity, the repeating unit itself derived from the specific polyfunctional compound may also contribute to the improvement of biocompatibility.

Presumably, due to the aforementioned mechanism of action, from the composition according to the embodiment of the present invention, a cured product and a film excellent in both the substrate adhesion and biocompatibility may be obtained.

The aforementioned substituted acrylamide group means, for example, an acrylamide group (for example, a methacrylamide group or the like) in which a carbon atom constituting a carbon-carbon double bond is substituted with an alkyl group. Furthermore, the aforementioned substituted acrylate group means an acrylate group (for example, a methacrylate group or the like) in which a carbon atom constituting a carbon-carbon double bond is substituted with an alkyl group.

Hereinafter, each of the components contained in the composition according to the embodiment of the present invention will be specifically described.

### <Specific polyfunctional compound>

The composition according to the embodiment of the present invention contains one or more kinds of specific polyfunctional compounds selected from the group consisting of a compound represented by Formula (1), a compound represented by Formula (2), and a compound represented by Formula (3). Particularly, in view of further improving the substrate adhesion, it is preferable that the composition according to the embodiment of the present invention contains the compound represented by Formula (3).

Hereinafter, the specific polyfunctional compound will be described.

### (Compound represented by Formula (1))

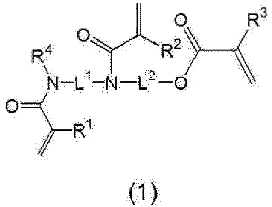

In Formula (1), R¹ to R⁴ each independently represent a hydrogen atom or an alkyl group.

The number of carbon atoms in the alkyl group represented by R¹ to R⁴ is not particularly limited, but is preferably 1 to 15, more preferably 1 to 10, even more preferably 1 to 6, and particularly preferably 1 to 3. The alkyl group may be linear, branched, or cyclic.

Examples of the alkyl group include a methyl group, an ethyl group, a n-propyl group, an i-propyl group, a n-butyl group, a t-butyl group, a n-hexyl group, a cyclopentyl group, a cyclohexyl group, and the like.

The alkyl group may have a substituent. The substituent that the alkyl group can have is not particularly limited, and examples thereof include a substituent W which will be described later.

As R¹ to R³, particularly, a hydrogen atom or an alkyl group having 1 to 6 carbon atoms is preferable, and a hydrogen atom or an alkyl group having 1 to 3 carbon atoms is more preferable.

As R⁴, a hydrogen atom is particularly preferable.

L¹ and L² each independently represent an alkylene group which may contain one or more kinds of divalent linking groups selected from the group consisting of -O-, -S-, -NR^{A}-, and -CO-.

The number of carbon atoms in the alkylene group represented by L¹ and L² is not particularly limited, but is, for example, preferably 1 to 15, more preferably 1 to 10, and even more preferably 1 to 4. In L¹ and L², it is preferable that a carbon atom is located at a position adjacent to a nitrogen atom in the amide group specified in the chemical formula and at a position adjacent to an oxygen atom (-O-) of the ester group specified in the chemical formula.

R^{A} represents a hydrogen atom or an alkyl group. The alkyl group represented by R^{A} has the same definition as the alkyl group represented by R¹ to R⁴ described above, and the suitable embodiments thereof are also the same. As R^{A}, a hydrogen atom is particularly preferable.

Particularly, in view of further improving the substrate adhesion, L¹ and L² are more preferably each independently an alkylene group having 1 to 4 carbon atoms or an alkylene group having 1 to 4 carbon atoms containing -O-.

Specific examples of the compound represented by Formula (1) will be shown below, but the present invention is not limited thereto.

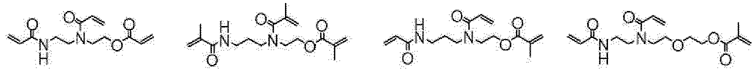

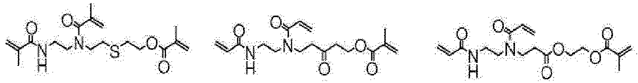

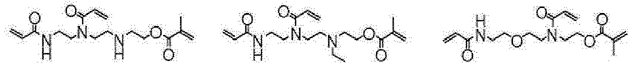

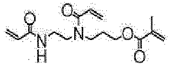

### (Compound represented by Formula (2))

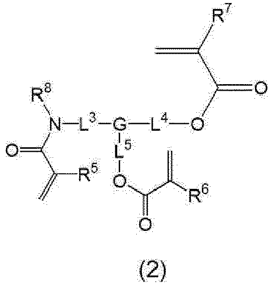

In Formula (2), R⁵ to R⁸ each independently represent a hydrogen atom or an alkyl group.

The alkyl group represented by R⁵ to R⁸ has the same definition as the alkyl group represented by R¹ to R⁴ in Formula (1) described above, and the suitable embodiments thereof are also the same.

The suitable embodiments of R⁵ to R⁷ are the same as the suitable embodiments of R¹ to R³ in Formula (1) described above.

The suitable embodiments of R⁸ are the same as the suitable embodiments of R⁴ in Formula (1) described above.

L³ represents a single bond or an alkylene group which may contain one or more kinds of divalent linking groups selected from the group consisting of -O-, -S-, -NR^{A}- and -CO-. In a case where L³ represents an alkylene group which may contain one or more kinds of divalent linking groups selected from the group consisting of -O-, -S-, -NR^{A}-, and -CO-, in L³, it is preferable that a carbon atom is located at a position adjacent to a nitrogen atom in the amide group specified in the chemical formula and a position adjacent to G specified in the chemical formula (provided that the position adjacent to L³ in G is other than a carbon atom).

L⁴ and L⁵ each independently represent an alkylene group which may contain one or more kinds of divalent linking groups selected from the group consisting of -O-, -S-, -NR^{A}-, and -CO-. In L⁴ and L⁵, it is preferable that a carbon atom is located at a position adjacent to an oxygen atom (-O-) in the ester group specified in the chemical formula and a position G specified in the chemical formula (provided that the position adjacent to L⁴ and L⁵ in G is other than a carbon atom).

The alkylene group, which is represented by L³ to L⁵ and may contain one or more kinds of divalent linking groups selected from the group consisting of -O-, -S-, -NR^{A-} and -CO-, has the same definition as the alkylene group which is represented by L¹ and L² in Formula (1) described above and may contain one or more kinds of divalent linking groups selected from the group consisting of -O-, -S-, -NR^{A}- and -CO-, and the suitable embodiments thereof are also the same.

Particularly, in view of further improving the substrate adhesion, L³ is more preferably a single bond, an alkylene group having 1 to 4 carbon atoms, or an alkylene group having 1 to 4 carbon atoms including -O-, or an alkylene group having 1 to 4 carbon atoms, and particularly preferably a single bond.

Particularly, in view of further improving the substrate adhesion, L⁴ and L⁵ more preferably each independently prepresent an alkylene group having 1 to 4 carbon atoms or an alkylene group having 1 to 4 carbon atoms containing -O-, and even more preferably each independently represent an alkylene group having 1 to 4 carbon atoms.

R^{A} represents a hydrogen atom or an alkyl group. The alkyl group represented by R^{A} has the same definition as the alkyl group represented by R¹ to R⁴ described above, and the suitable embodiments thereof are also the same. As R^{A}, a hydrogen atom is particularly preferable.

G represents a trivalent linking group. G is, for example, preferably a group represented by Formula (Y1), a nitrogen atom, a group represented by Formula (Y3), a trivalent aliphatic heterocyclic group (preferably, a group represented by Formula (Y4) or a group represented by Formula (Y5)), or a trivalent aliphatic hydrocarbon ring (preferably a group represented by Formula (Y6)).

In a case where G represents "nitrogen atom", G is a group represented by Formula (Y2).

In each of the general formulas, * represents a position linked to L³, L⁴, and L⁵.

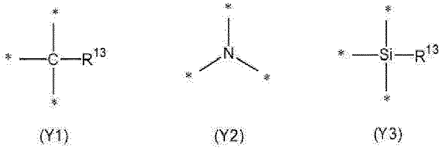

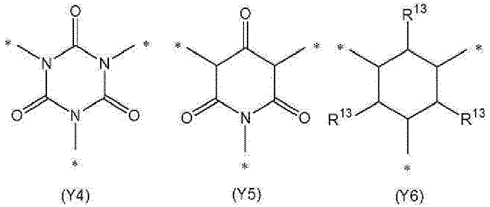

The number of carbon atoms contained in the aliphatic hydrocarbon ring is preferably 3 to 15, more preferably 3 to 10, and even more preferably 5 to 10. The aliphatic heterocyclic ring is preferably a 5- to 7-membered ring having at least one N, O, S, or Se atom in the ring structure, more preferably a 5- to 6-membered ring.

In the groups represented by Formulas (Y1) to (Y6) described above, R¹³ represents a hydrogen atom or a substituent. The substituent represented by R¹³ is not particularly limited, and examples thereof include a substituent W which will be described later. Particularly, as the substituent represented by R¹³, an alkyl group (any of a linear, branched, or cyclic alkyl group preferably having 1 to 10 carbon atoms and more preferably having 1 to 6 carbon atoms) or a group represented by Formula (Z) is preferable.

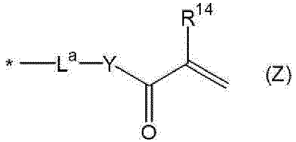

In Formula (Z), R¹⁴ represents a hydrogen atom or an alkyl group.

The alkyl group represented by R¹⁴ has the same definition as the alkyl group represented by R¹ to R⁴ in Formula (1) described above, and the suitable embodiments thereof are also the same. The suitable embodiments of R¹⁴ are the same as the suitable embodiments of R¹ to R³ in Formula (1) described above.

Y represents -O- or -NR^{A}-. R^{A} represents a hydrogen atom or an alkyl group. The alkyl group represented by R^{A} has the same definition as the alkyl group represented by R¹ to R⁴ described above, and the suitable embodiments thereof are also the same. As R^{A}, a hydrogen atom is particularly preferable.

L^{a} represents a single bond or a divalent linking group. The divalent linking group is not particularly limited, and examples thereof include -O-, an alkylene group having 1 to 4 carbon atoms, and a divalent linking group obtained by combining these. In the divalent linking group represented by L^{a}, a carbon atom is usually located at a position adjacent to Y

Examples of the "divalent linking group obtained by combining these" include an alkylene group having 1 to 4 carbon atoms containing -O-, such as -OCH₂-, -OCH₂CH₂-, -OCH₂CH₂CH₂-, -OCH₂CH₂CH₂CH₂-, -CH₂OCH₂-, -CH₂OCH₂CH₂-, or -CH₂OCH₂CH₂CH₂-, and a group represented by -(O-alkylene group (having 1 to 4 carbon atoms))ₙ-. Herein, n represents an integer of 2 or greater. The upper limit of n is, for example, about 10.

Particularly, in view of further improving the substrate adhesion, L^{a} more preferably each independently represents an alkylene group having 1 to 4 carbon atoms or an alkylene group having 1 to 4 carbon atoms containing -O-, and even more preferably each independently represents an akylene groups having 1 to 4 carbon atoms.

Specific examples of the compound represented by Formula (2) will be shown below, but the present invention is not limited thereto.

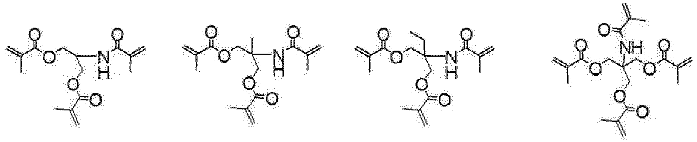

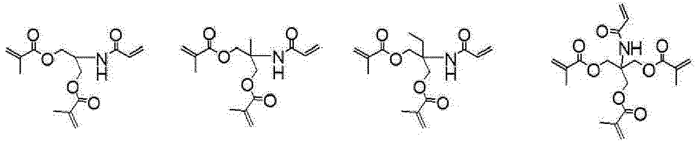

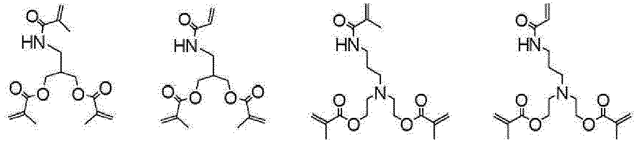

### (Compound represented by Formula (3))

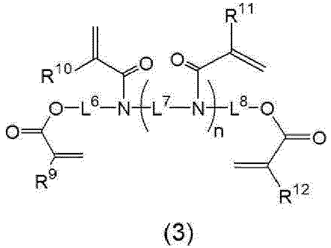

In Formula (3), R⁹ to R¹² each independently represent a hydrogen atom or an alkyl group.

The alkyl group represented by R⁹ to R¹² has the same definition as the alkyl group represented by R¹ to R⁴ in Formula (1) described above, and the suitable embodiments thereof are also the same. The suitable embodiments of R⁹ to R¹² are the same as the suitable embodiments of R¹ to R³ in Formula (1) described above.

L⁶ to L⁸ each independently represent an alkylene group which may contain one or more kinds of divalent linking groups selected from the group consisting of -O-, -S-, -NR^{A}-, and -CO-. The alkylene group, which is represented by L⁶ to L⁸ and may contain one or more kinds of divalent linking groups selected from the group consisting of -O-, -S-, -NR^{A}-, and -CO-, has the same definition as the alkylene group which is represented by L¹ and L² in Formula (1) described above and may contain one or more kinds of divalent linking groups selected from the group consisting of -O-, -S-, -NR^{A}- and -CO-, and the suitable embodiments thereof are also the same. In L⁶ and L⁸, it is preferable that a carbon atom is located at a position adjacent to a nitrogen atom in the amide group specified in the chemical formula and a position adjacent to an oxygen atom (-O-) in the ester group specified in the chemical formula. In L⁷, it is preferable that a carbon atom is located at a position adjacent to a nitrogen atom in the amide group specified in the chemical formula.

Particularly, in view of further improving the substrate adhesion, L⁶ to L⁸ more preferably each independently represent an alkylene group having 1 to 4 carbon atoms or an alkylene group having 1 to 4 carbon atoms containing -O-, and even more preferably each independently represent an alkylene group having 1 to 4 carbon atoms.

R^{A} represents a hydrogen atom or an alkyl group. The alkyl group represented by R^{A} has the same definition as the alkyl group represented by R¹ to R⁴ described above, and the suitable embodiments thereof are also the same. As R^{A}, a hydrogen atom is particularly preferable.

n represents an integer of 0 to 3. In view of further improving the substrate adhesion, n is preferably 0 or 1. In a case where n represents an integer equal to or greater than 2, a plurality of L⁷s may be the same as or different from each other, and a plurality of R¹¹s may be the same as or different from each other.

Specific examples of the compound represented by Formula (3) will be shown below, but the present invention is not limited thereto.

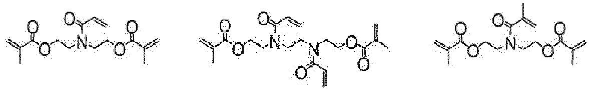

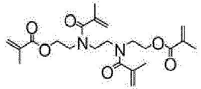

The specific polyfunctional compound can be synthesized by a known method.

One kind of the specific polyfunctional compound may be used singly, or two or more kinds of the specific polyfunctional compounds may be used in combination.

In the composition according to the embodiment of the present invention, the content of the specific polyfunctional compound (total content in a case where the composition contains a plurality of kinds of the specific polyfunctional compounds) with respect to the total solid content of the composition is preferably 10% to 90% by mass, more preferably 20% to 80% by mass, and even more preferably 30% to 70% by mass. In the present specification, "solid content" means components constituting a film (cured film), and do not include a solvent. A monomer is a component constituting the cured film. Therefore, the monomer is included in the solid content even if the monomer is a liquid.

### <Specific polyfunctional (meth)acrylamide compound or betaine monomer>

The composition according to the embodiment of the present invention contains one or more kinds of compounds selected from the group consisting of a specific polyfunctional (meth)acrylamide compound and a betaine monomer. In view of further improving the substrate adhesion, the composition according to the embodiment of the present invention preferably contains a specific polyfunctional (meth)acrylamide compound. In view of further improving both the substrate adhesion and biocompatibility, the composition according to the embodiment of the present invention more preferably contains both the polyfunctional (meth)acrylamide compound and betaine monomer. That is, it is more preferable that the specific polyfunctional compound, the specific polyfunctional (meth)acrylamide compound, and the betaine monomer are used in combination.

Hereinafter, the specific polyfunctional (meth)acrylamide compound and the betaine monomer will be described respectively.

### (Specific polyfunctional (meth)acrylamide compound (compound represented by Formula (A)))

The specific polyfunctional (meth)acrylamide compound is a compound represented by Formula (A).

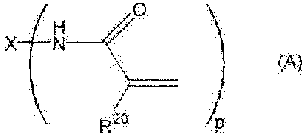

In Formula (A), R²⁰ represents a hydrogen atom or a methyl group. A plurality of R²⁰s may be the same as or different from each other.

X represents a p-valent linking group. p represents an integer of 2 to 4.

X is not particularly limited, and examples thereof include an alkylene group which may contain one or more kinds of divalent linking groups selected from the group consisting of -O-, -S-, -NR^{A}-, -CO-, and Formula (B) and groups represented by Formula (Z1) and Formula (Z2). The number of carbon atoms in the alkylene group is not particularly limited, but is, for example, 1 to 100. R^{A} represents a hydrogen atom or an alkyl group. The alkyl group represented by R^{A} has the same definition as the alkyl group represented by R¹ to R⁴ in Formula (1) described above, and the suitable embodiments thereof are also the same. As R^{A}, a hydrogen atom is particularly preferable.

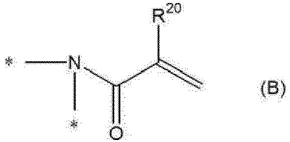

In Formula (B), R²⁰ represents a hydrogen atom or a methyl group. In addition, * represents a linking position.

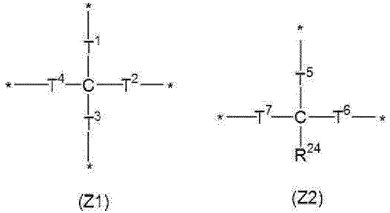

In Formulas (Z1) and (Z2), T¹ to T⁷ each independently represent a single bond or a divalent linking group. The divalent linking group is not particularly limited, and examples thereof include an alkylene group having 1 to 10 carbon atoms that may contain one or more kinds of divalent linking groups selected from the group consisting of -O-, -S-, -NR^{A}-, and -CO-. Particularly, in view of further improving the substrate adhesion, T¹ to T⁷ more preferably each independently represent an alkylene group having 2 to 4 carbon atoms containing -O-. In T¹ to T⁷, it is preferable that a carbon atom is located at a position adjacent to a nitrogen atom in the amide group specified in Formula (A).

In Formula (Z2), R²⁴ represents a hydrogen atom or a substituent. The substituent represented by R²⁴ is not particularly limited, and examples thereof include the substituent W which will be described later. The substituent represented by R²⁴ particularly preferably an alkyl group (any of a linear, branched, or cyclic alkyl group preferably having 1 to 10 carbon atoms and more preferably having 1 to 6 carbon atoms). R²⁴ is preferably a hydrogen atom or an alkyl group (for example, an alkyl group having 1 to 6 carbon atoms and preferably having 1 to 3 carbon atoms).

In view of further improving the biocompatibility, the compound represented by Formula (A) described above is preferably a compound represented by Formula (A1) or a compound represented by Formula (A2).

### .Compound represented by Formula (A1)

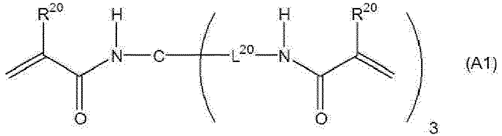

In Formula (A1), R²⁰ each independently represents a hydrogen atom or a methyl group. A plurality of R ²⁰s may be the same as or different from each other.

L²⁰ each independently represents -O-, an alkylene group having 2 to 4 carbon atoms, or a divalent linking group obtained by combining these. In L²⁰, it is preferable that a carbon atom is located at a position adjacent to a nitrogen atom in the amide group specified in the chemical formula. That is, as the group adjacent to a nitrogen atom in the amide group, an alkylene group having 2 to 4 carbon atoms is preferably located at the aforementioned position.

Examples of the aforementioned "divalent linking group obtained by combining these" include an alkylene group having 2 to 4 carbon atoms containing -O-, such as -OCH₂CH₂-, -OCH₂CH₂CH₂-, -OCH₂CH₂CH₂CH₂-, -CH₂OCH₂-, -CH₂OCH₂CH₂-, or -CH₂OCH₂CH₂CH₂- and a group represented by -(O-alkylene group (having 2 to 4 carbon atoms))ₙ-, and the like. Herein, n represents an integer of 2 or greater. The upper limit of n is not particularly limited, but is, for example, about 10.

In each of the groups exemplified as "divalent linking group obtained by combining these", any of the two binding sites may be bonded to the amide group.

Particularly, in view of further improving the substrate adhesion and the biocompatibility, L²⁰ is preferably an alkylene group having 2 to 4 carbon atoms containing -O-.

Furthermore, a plurality of L²⁰s may be the same as or different from each other.

### .Compound represented by Formula (A2)

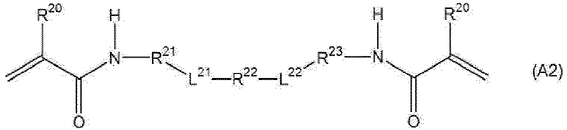

In Formula (A2), R²⁰ each independently represents a hydrogen atom or a methyl group.

R²¹ and R²³ each independently represent -O-, an alkylene group having 1 to 4 carbon atoms, or a divalent linking group obtained by combining these. In R²¹ and R²³, it is preferable that a carbon atom is usually located at a position adjacent to a nitrogen atom in the amide group specified in the chemical formula. As the group adjacent to a nitrogen atom in the amide group, an alkylene group having 1 to 4 carbon atoms is preferably located at the aforementioned position.

Examples of the "divalent linking group obtained by combining these" include an alkylene group having 1 to 4 carbon atoms containing -O-, such as -OCH₂-, -OCH₂CH₂-, -OCH₂CH₂CH₂-, -OCH₂CH₂CH₂CH₂-, -CH₂OCH₂-, -CH₂OCH₂CH₂-, or -CH₂OCH₂CH₂CH₂-, and a group represented by -(O-alkylene group (having 1 to 4 carbon atoms))ₙ-. Herein, n represents an integer of 2 or greater. The upper limit of n is not particularly limited, but is, for example, about 10.

In each of the groups exemplified as "divalent linking group obtained by combining these", any of the two binding sites may be bonded to the amide group.

Particularly, in view of further improving the substrate adhesion and the biocompatibility, R²¹ and R²³ more preferably each independently represent an alkylene group having 1 to 4 carbon atoms or an alkylene group having 1 to 4 carbon atoms containing -O-.

In Formula (A2), R²² represents -O-, an alkylene group having 1 to 4 carbon atoms, a group represented by Formula (B), or a divalent linking group obtained by combining these.

Examples of the "divalent linking group obtained by combining these" include the groups described above for R²¹ and R²³. In a case where the group represented by Formula (B) is combined with another group, it is preferable that an alkylene group having 1 to 4 carbon atoms is bonded to a nitrogen atom in the group represented by Formula (B).

Particularly, in view of further improving the substrate adhesion and the biocompatibility, R²² is more preferably an alkylene group having 1 to 4 carbon atoms, an alkylene group having 1 to 4 carbon atoms containing -O-, or the group represented by Formula (B).

L²¹ and L²² each independently represent a single bond or a group represented by Formula (B).

In a case where R²² represents Formula (B), it is preferable that both the L²¹ and L²² represent a single bond.

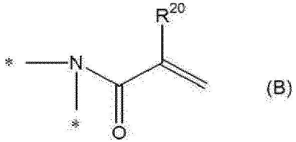

In Formula (B), R²⁰ represents a hydrogen atom or a methyl group, and * represents a linking position. Usually, * is linked to a carbon atom.

Specific examples of the specific polyfunctional (meth)acrylamide compound will be shown below, but the present invention is not limited thereto.

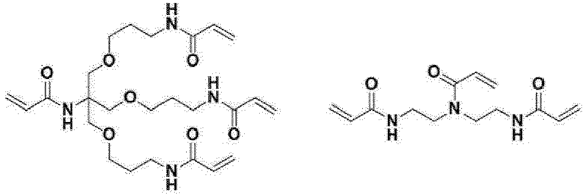

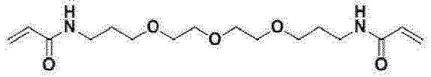

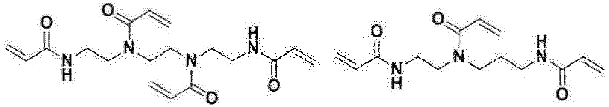

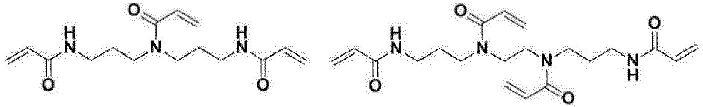

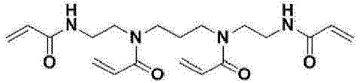

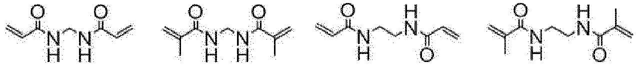

As the specific polyfunctional (meth)acrylamide compound, various commercially available products can be used. Alternatively, the specific polyfunctional (meth)acrylamide compound can be synthesized by the method described in Journal of Technical Disclosure No. 2013-502654.

One kind of the specific polyfunctional (meth)acrylamide compound may be used singly, or two or more kinds of the specific polyfunctional (meth)acrylamide compounds may be used in combination.

In the composition according to the embodiment of the present invention, the content of the specific polyfunctional (meth)acrylamide compound (total content in a case where the composition contains a plurality of kinds of the specific polyfunctional (meth)acrylamide compounds) with respect to the total solid content of the composition is preferably 10% to 90% by mass, more preferably 20% to 80% by mass, even more preferably 30% to 60% by mass, and particularly preferably 30% to 55% by mass.

### (Betaine monomer)

The betaine monomer that the composition according to the embodiment of the present invention can contain is not particularly limited. Examples of the betaine monomer include a monomer having a betaine structure such as a sulfobetaine structure, a phosphobetaine structure, or a carboxybetaine structure. Generally, betaine refers to a compound (inner salt) which has a positive charge and a negative charge at non-adjacent positions in the same molecule but does not carry a charge as a whole molecule, in which a hydrogen atom is not bonded to an atom having a positive charge.

The skeleton of the betaine monomer that the composition according to the embodiment of the present invention can contain is not particularly limited, but is preferably an acrylate-based monomer or an acrylamide-based monomer is preferable.

Particularly, in view of further improving the biocompatibility, as the betaine monomer that the composition according to the embodiment of the present invention can contain, a compound represented by Formula (C) is preferable.

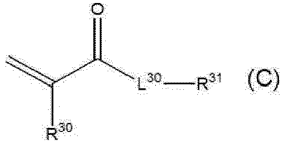

### In Formula (C), R³⁰ represents a hydrogen atom or an alkyl group.

The alkyl group represented by R³⁰ has the same definition as the alkyl group represented by R¹ to R⁴ in Formula (1) described above, and the suitable embodiments thereof are also the same. Furthermore, the suitable embodiments of R³⁰ are the same as the suitable embodiments of R¹ to R³ in Formula (1) described above.

L³⁰ represents -O- or -NR^{A}-.

R^{A} represents a hydrogen atom or an alkyl group. The alkyl group represented by R^{A} has the same definition as the alkyl group represented by R¹ to R⁴ in Formula (1) described above, and the suitable embodiments thereof are also the same. As R^{A}, a hydrogen atom is particularly preferable.

R³¹ represents a monovalent group represented by Formula (I), a monovalent group represented by Formula (II), or a monovalent group represented by Formula (III).

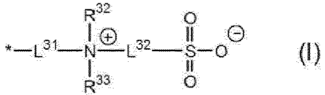

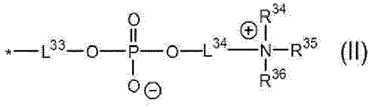

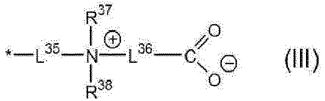

In Formula (I), L³¹ and L³² each independently represent a divalent linking group.

L³¹ and L³² are not particularly limited, but may be an alkylene group having 1 to 10 carbon atoms that may contain a heteroatom (the alkylene group may be any of a linear, branched, or cyclic alkylene group, but is preferably a linear alkylene group). The number of carbon atoms in the alkylene group is more preferably 1 to 6, even more preferably 1 to 4, and particularly preferably 2 to 4.

R³² and R³³ each independently represent an alkyl group.

The number of carbon atoms in the alkyl group represented by R³² and R³³ is not particularly limited, but is preferably 1 to 6 and more preferably 1 to 3. The alkyl group may be linear, branched, or cyclic.

Examples of the alkyl group include a methyl group, an ethyl group, a n-propyl group, and an i-propyl group.

The alkyl group may have a substituent. The substituent that the alkyl group can have is not particularly limited, and examples thereof include a substituent W which will be described later.

* represents a binding position.

In Formula (II), L³³ and L³⁴ each independently represent a divalent linking group.

The divalent linking group represented by L³³ and L³⁴ has the same definition as the divalent linking group represented by L³¹ and L³² in Formula (I) described above, and the suitable embodiments thereof are also the same.

R³⁴ to R³⁶ each independently represent an alkyl group.

The alkyl group represented by R³⁴ to R³⁶ has the same definition as the alkyl group represented by R³² and R³³ in Formula (I) described above, and the suitable embodiments thereof are also the same.

* represents a binding position.

In Formula (III), L³⁵ and L³⁶ each independently represent a divalent linking group.

The divalent linking group represented by L³⁵ and L³⁶ has the same definition as the divalent linking group represented by L³¹ and L³² in Formula (I) described above, and the suitable embodiments thereof are also the same.

R³⁷ and R³⁸ each independently represent an alkyl group.

The alkyl group represented by R³⁷ and R³⁸ has the same definition as the alkyl group represented by R³² and R³³ in Formula (I) described above, and the suitable embodiments thereof are also the same.

* represents a binding position.

Particularly, in view of further improving the biocompatibility, as R³¹, any of the group represented by Formula (I) or the group represented by Formula (II) is preferable.

The betaine monomer can be synthesized by a known method.

One kind of the betaine monomer may be used singly, or two or more kinds of the betaine monomers may be used in combination.

In the composition according to the embodiment of the present invention, the content of the betaine monomer (total content in a case where the composition contains a plurality of kinds of the betaine monomers) with respect to the total solid content of the composition is preferably 10% to 50% by mass, more preferably 10% to 45% by mass, and even more preferably 15% to 40% by mass.

Specific examples of the betaine monomer will be shown below, but the present invention is not limited thereto.

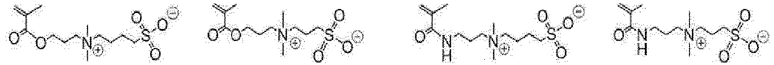

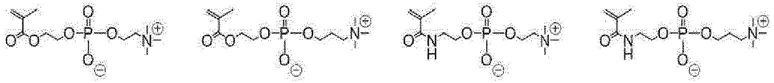

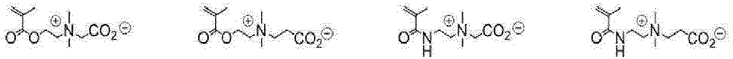

### (Substituent group W)

Examples of the substituent W includes an alkyl group (preferably an alkyl group having 1 to 20 carbon atoms), an alkenyl group (preferably an alkenyl group having 2 to 20 carbon atoms), an alkynyl group (preferably an alkynyl group having 2 to 20 carbon atoms), a cycloalkyl group (preferably a cycloalkyl group having 3 to 20 carbon atoms), an aryl group (preferably an aryl group having 6 to 26 carbon atoms), a heterocyclic group (preferably a heterocyclic group having 2 to 20 carbon atoms and more preferably a 5- or 6-membered heterocyclic group having at least one oxygen atom, sulfur atom, or nitrogen atom), an alkoxy group (preferably an alkoxy group having 1 to 20 carbon atoms), an aryloxy group (preferably an aryloxy group having 6 to 26 carbon atoms), an alkoxycarbonyl group (preferably an alkoxycarbonyl group having 2 to 20 carbon atoms),an aryloxycarbonyl group (preferably an aryloxycarbonyl group having 6 to 26 carbon atoms), an amino group (preferably an amino group having 0 to 20 carbon atoms including an alkylamino group and an arylamino group, such as amino, N,N-dimethylamino, N,N-diethylamino, N-ethylamino, and anilino), a sulfamoyl group (preferably a sulfamoyl group having 0 to 20 carbon atoms), an acyl group (preferably an acyl group having 1 to 20 carbon atoms), an acyloxy group (preferably an acyloxy group having 1 to 20 carbon atoms), a carbamoyl group (preferably a carbamoyl group having 1 to 20 carbon atoms), an acylamino group (preferably an acylamino group having 1 to 20 carbon atoms, such as an acetylamino group or a benzoylamino group), an alkylthio group (preferably an alkylthio group having 1 to 20 carbon atoms), an arylthio group (preferably an arylthio group having 6 to 26 carbon atoms), an alkylsulfonyl group (preferably an alkylsulfonyl group having 1 to 20 carbon atoms), an arylsulfonyl group (preferably an arylsulfonyl group having 6 to 22 carbon atoms), an alkylsilyl group (preferably an alkylsilyl group having 1 to 20 carbon atoms), an arylsilyl group (preferably an arylsilyl group having 6 to 42 carbon atoms), an alkoxysilyl group (preferably an alkoxysilyl group having 1 to 20 carbon atoms), an aryloxysilyl group (preferably an aryloxysilyl group having 6 to 42 carbon atoms), a phosphoryl group (preferably a phosphoryl group having 0 to 20 carbon atoms, for example, -OP(=O)(R^{P})₂), a phosphonyl group (preferably a phosphonyl group having 0 to 20 carbon atoms, for example, -P(=O)(R^{P})₂), a phosphinyl group (preferably a phosphinyl group having 0 to 20 carbon atoms, for example, -P(R^{P})₂), a (meth)acryloyl group, a (meth)acryloyloxy group, a (meth)acryloylimino group (a (meth)acrylamide group), a hydroxyl group, a thiol group, a carboxyl group, a phosphoric acid group, a phosphonic acid group, a sulfonic acid group, a cyano group, and a halogen atom (for example, a fluorine atom, a chlorine atom,a bromine atom, an iodine atom, or the like). R^{P} represents a hydrogen atom, a hydroxyl group, or a substituent.

In addition, each of the groups exemplified as the substituent W may be further substituted with the substituent W.

In a case where the aforementioned substituent is an acidic group or a basic group, the substituent may form a salt thereof.

In a case where the compound, the substituent, the linking group, and the like contain an alkyl group, an alkylene group, an alkenyl group, an alkenylene group, an alkynyl group, an alkynylene group, or the like, these may be cyclic or linear or may be linear or branched, and may be substituted as described above or unsubstituted.

### <Initiator>

It is preferable that the composition according to the embodiment of the present invention contains an initiator.

The initiator is not particularly limited, but is preferably a thermal polymerization initiator or a photopolymerization initiator.

Examples of the photopolymerization initiator include an alkylphenone-based photopolymerization initiator, a methoxyketone-based photopolymerization initiator, an acylphosphine oxide-based photopolymerization initiator, a hydroxyketone-based photopolymerization initiator (for example, IRGACURE184; 1,2-α-hydroxyalkylphenone), an aminoketone-based photopolymerization initiator (for example, 2-methyl-1-[4-(methylthio)phenyl]-2-morpholino-propan-1-one (IRGACURE (registered trademark) 907)), an oxime-based photopolymerization initiator, and an oxyphenylacetic acid ester-based photopolymerization initiator (IRGACURE (registered trademark) 754), and the like.

Examples of other initiators include an azo-based polymerization initiator (for example, V-50 or V-601), a persulfate-based polymerization initiator, a persulfuric acid-based polymerization initiator, a redox-based polymerization initiator, and the like.

One kind of the initiator may be used singly, or two or more kinds of the initiators may be used in combination.

In the composition according to the embodiment of the present invention, the content of the initiator (total content in a case where the composition contains a plurality of kinds of initiators) is not particularly limited. However, the content of the initiator with respect to the total solid content of the composition is preferably 0.5% to 10% by mass, and more preferably 1% to 5% by mass.

### <Solvent>

It is preferable that the composition according to the embodiment of the present invention contains a solvent.

Examples of the solvent include water, an organic solvent (for example, esters such as ethyl acetate and n-butyl acetate; aromatic hydrocarbons such as toluene and benzene; aliphatic hydrocarbons such as n-hexane and n-heptane; alicyclic hydrocarbons such as cyclohexane and methylcyclohexane; ketones such as methyl ethyl ketone (MEK), methyl isobutyl ketone, and cyclohexanone; alcohols such as methanol and butanol, or the like), and a mixed solvent of these.

Among these, from the viewpoint of making it difficult for surface unevenness to occur during coating, alcohol solvents such as methanol and ethanol are preferable.

One kind of the solvent may be used singly, or two or more kinds of the solvents may be used in combination.

In the composition according to the embodiment of the present invention, the content of the solvent (total content in a case where the composition contains a plurality of kinds of solvents) with respect to the total mass of the composition is preferably from 0.5% to 95% by mass, more preferably 1% to 90% by mass, and even more preferably 10% to 80% by mass.

### <Other components>

The composition according to the embodiment of the present invention may contain components other than the components described above. Examples of such components include a binder resin, a polyfunctional amine (this polyfunctional amine is other than the specific polyfunctional compound and the specific polyfunctional (meth)acrylamide compound described above), a polyfunctional thiol, a surfactant, a plasticizer, and a surface lubricant, a leveling agent, a softener, an antioxidant, an antiaging agent, a light stabilizer, an ultraviolet absorber, an inorganic or organic filler, a metal powder, and the like.

The binder resin is not particularly limited, and examples thereof include an acrylic resin, a styrene-based resin, a vinyl-based resin, a polyolefin-based resin, a polyester-based resin, a polyurethane-based resin, a polyamide-based resin, a polycarbonate-based resin, a polydiene-based resin, an epoxy-based resin, a silicone-based resin, a cellulose-based polymer, a chitosan-based polymer, and the like.

### <Method for preparing curable composition>

As the method for preparing the composition according to the embodiment of the present invention, a known method can be employed without particular limitation. For example, the curable composition can be prepared by mixing together the above components and then stirring the mixture by known means.

### [Cured product]

The cured product according to an embodiment of the present invention is formed by curing the aforementioned composition according to the embodiment of the present invention. The shape of the cured product can be appropriately selected according to the use. Examples of the shape of the cured product include a powder shape and a film shape. Among these, a film shape is preferable.

In a case where the cured product is formed into a film, the film thickness is not particularly limited, but is, for example, 0.1 to 300 µm and more preferably 1 to 100 µm.

In addition, the cured product according to the embodiment of the present invention contains a polymer compound containing one or more kinds of repeating units selected from the group consisting of a repeating unit derived from a specific polyfunctional compound, a repeating unit derived from a specific polyfunctional (meth)acrylamide compound, and a repeating unit derived from a betaine monomer.

### <Method for manufacturing cured product (cured film)>

The method for manufacturing the cured product (cured film) according to the embodiment of the present invention is not particularly limited. Examples thereof include a method of coating a substrate with the aforementioned composition according to the embodiment of the present invention and then curing the composition by heating or light irradiation (examples of the light include ultraviolet rays, visible rays, X-rays, and the like).

The material of the substrate is not particularly limited, and examples thereof include a metal material, a ceramic material, a plastic material, and the like.

Examples of the type of the plastic material include polyethylene terephthalate, polybutylene terephthalate, polyethylene naphthalate, polyethylene, polypropylene, cellophane, diacetyl cellulose, triacetyl cellulose, acetyl cellulose butyrate, polyvinyl chloride, polyvinylidene chloride, polyvinyl alcohol, an ethylene-vinyl acetate copolymer, polystyrene, polycarbonate, polymethylpentene, polysulfone, polyether ether ketone, polyether sulfone, polyether imide, polyimide, a fluororesin, an acrylic resin, polyamide, cycloolefin, nylon, polyether sulfan, and the like.

Examples of the type of the metal material include gold, stainless steel, a cobalt-chromium alloy, an amalgam alloy, a silver-palladium alloy, a gold-silver-palladium alloy, titanium, a nickel-titanium alloy, platinum, and the like.

Examples of the type of the ceramic material include hydroxyapatite and the like.

The shape of the substrate is not particularly limited, and may be a plate shape or a three-dimensional shape.

Examples of the method for coating the substrate with the composition according to the embodiment of the present invention include methods such as roll coating, kiss roll coating, gravure coating, reverse coating, roll brush coating, spray coating, dip roll coating, bar coating, spin coating, knife coating, air knife coating, curtain coating, lip coating, and an extrusion coating method using a die coater.

The heating method is not particularly limited, and examples thereof include a method using a blast dryer, an oven, an infrared dryer, a heating drum, and the like.

The heating temperature is not particularly limited, but is preferably 30°C to 150°C and more preferably 40°C to 120°C.

The heating time is not particularly limited, but is usually 1 minute to 6 hours. In a case where the composition is dried in a coating apparatus, the heating time is 1 to 20 minutes, and the heating temperature at the time of heating after the coating (for example, heating the substrate that is wound up) is preferably room temperature to 50°C.

Examples of the method of light irradiation include methods using a low-pressure mercury lamp, a medium-pressure mercury lamp, a high-pressure mercury lamp, a metal halide lamp, a deep-UV (ultraviolet) light, an LED (light emitting diode) lamp, a xenon lamp, a chemical lamp, a carbon arc lamp, and the like. The energy of light irradiation is not particularly limited, but is preferably 0.1 to 10 J/cm².

### <Use>

The cured product according to the embodiment of the present invention exhibits excellent substrate adhesion and can inhibit or prevent the adhesion of biological materials such as cells and blood components. Therefore, the cured product according to the embodiment of the present invention is suitably used for prostheses, medical instruments, and the like as a material applied to a living body (biomaterial). Specifically, the cured product according to the embodiment of the present invention may be used as a filler for a resin composition used as a material for a prosthesis, a medical instrument, and the like, or may be used as a coating material by being disposed on the surface of a prosthesis or a medical instrument. Examples of the medical instrument include a denture, an artificial dialysis membrane, a catheter, and the like. The prosthesis refers to a member that is incorporated into the human body for a long-term treatment or the like, and examples thereof include an artificial blood vessel, a stent, an artificial organ, an artificial bone, an artificial valve, cultured skin, and the like.

The cured product according to the embodiment of the present invention is particularly preferably used as a dental material or an artificial bone adhesive.

### [Medical member]

The medical member according to an embodiment of the present invention includes a substrate and a cured product disposed on the substrate.

The substrate means the aforementioned prosthesis, medical instrument, and the like, and examples thereof include those made of the metal material, the ceramic material, and the plastic material described above. Specifically, examples of the substrate include dentures and artificial bones.

The cured film corresponds to the aforementioned cured product having a film shape. Examples

Hereinafter, the present invention will be more specifically described based on examples. The materials, the amount and ratio thereof used, how to treat the materials, the treatment procedure, and the like described in the following examples can be appropriately changed as long as the gist of the present invention is maintained. Therefore, the scope of the present invention is not limited to the following examples.

### [Components of curable composition]

### <Specific polyfunctional compound (compounds represented by Formula (1) to Formula (3)>

ex (1) to ex (8) shown in Table 1 will be illustrated below.

ex (1) and ex (2) correspond to the compound represented by Formula (1), ex (3) and ex (4) correspond to the compound represented by Formula (2), and ex (5) to ex (8) correspond to the compound represented by Formula (3).

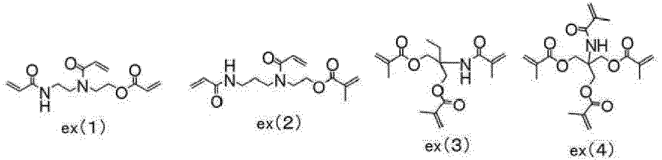

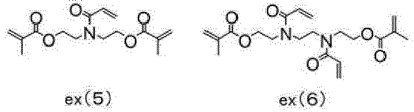

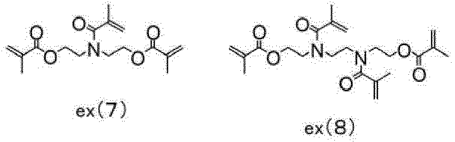

### (Synthesis of ex (1) to ex (8))

ex (1) to ex (8) were synthesized according to the synthesis method described in paragraph "0107" in JP2013-053082A.

### <Specific polyfunctional (meth)acrylamide compound (compound represented by Formula (A))>

B (1) to B (5) shown in Table 1 will be illustrated below.

B (1) corresponds to the compound represented by Formula (A1), and B(2) to B (4) correspond to the compound represented by Formula (A2).

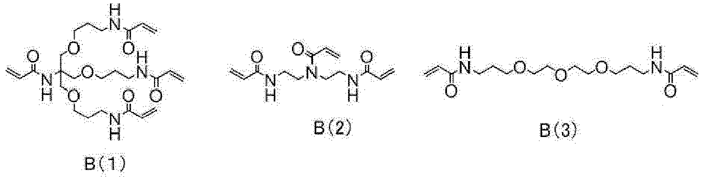

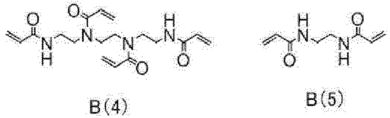

### (Synthesis of B (1))

B (1) was synthesized according to the synthesis of the polymerizable compound 1 in the Journal of Technical Disclosure No. 2013-502654 of Japan Institute for Promoting Invention and Innovation.

### (Synthesis of B (2) to B (5))

According to the synthesis of the polyfunctional compound 9, the polyfunctional compound 8, the polyfunctional compound 10, and the polyfunctional compound 2 of the Journal of Technical Disclosure No. 2013-502654 of Japan Institute for Promoting Invention and Innovation, B (2) to B (5) were synthesized respectively.

### <Betaine monomer (compound represented by Formula (C))>

C (1) to C (3) shown in Table 1 will be shown below.

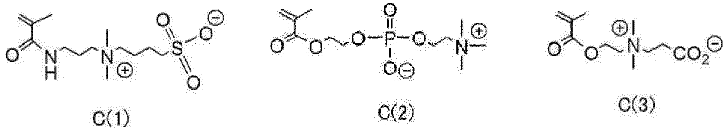

### (Synthesis of C (1) to C (3))

C (1) was synthesized according to the procedure described in WO2016/067795A. As C (2) and C (3), the compounds purchased from TOKYO CHEMICAL INDUSTRY CO., LTD. were used.

### <Comparative polyfunctional compound>

Com (1) and Com (2) shown in Table 1 will be shown below.

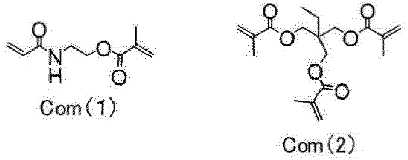

### (Synthesis of Com (1))

Com (1) was synthesized according to the description in paragraph 0075 in JP2014-010490A.

### (Synthesis of Com (2))

As Com (2), the compound purchased from Sigma-Aldrich Co. LLC. was used.

### [Preparation of curable composition]

The components shown in the following Table 1 were dissolved in a solvent (methanol), thereby preparing curable compositions having a concentration of solid contents of 20% by mass (curable compositions 1 to 34). Regarding the curable compositions, the solid contents mean all components except for the solvent.

The numerical value in Table 1 represents the content (% by mass) of each component with respect to the total solid content of a curable composition. "Irg2959" corresponds to a polymerization initiator ("IRGACURE 2959", manufactured by BASF SE).

### [Preparation of film]

### <Preparation of coat film for evaluating adhesion and biocompatibility>

By using a spin coater, a substrate (acrylic plate (manufacturer: MISUMI Corporation, model number: ACA)) was coated with each of the prepared curable compositions such that the film thickness became about 5 µm, and then the composition was dried. Then, by using an "ECS-401G (trade name)" UV (ultraviolet) exposure machine (light source: high-pressure mercury lamp) manufactured by EYE GRAPHICS Co., Ltd., the composition was exposed at an exposure amount of 4 J/cm², thereby preparing a coat film for evaluating adhesion and biocompatibility.

### [Evaluation]

### <Substrate adhesion test>

The prepared acrylic plate with a coat film was immersed in a PBS (Phosphate buffered saline) solution at 37°C for 24 hours. Thereafter, the acrylic plate with a coat film was pulled up from the solution, and the substrate adhesion was evaluated based on the area of the coat film remaining on the acrylic plate (hereinafter, referred to as "residual coat film" as well). The area of the residual coat film with respect to the area of the acrylic plate was expressed as a percentage as a coating rate, and evaluated based on the following evaluation standard. Regarding the evaluation of the substrate adhesion, samples graded "B" or higher were regarded as acceptable. The results are shown in Table 1.

### (Evaluation standard)

"A": The coating rate was equal to or higher than 90%.
"B": The coating rate was equal to or higher than 70% and less than 90%.
"C": The coating rate was equal to or higher than 50% and less than 70%.
"D": The coating rate was less than 50%.

### <Evaluation of biocompatibility (cell adhesion test)>

The prepared acrylic substrate with a coat film was placed in a 6-well plate, and mouse-derived fibroblasts (3T3 cells) were dispersed in Dulbecco's modified Eagle medium at a seeding density of 1.0 × 10⁵ cells/cm². By using an incubator, the cells were cultured for 24 hours under the condition of 37°C and 5% carbon dioxide.

Thereafter, the acrylic substrate with the coat film was taken out observed using a phase contrast microscope (an inverted cubic research microscope, manufactured by Olympus Corporation) so as to check whether or not the cells were attached thereto. The magnification was 4X.

This operation was performed on 10 acrylic substrates with a coat film, and biocompatibility was evaluated as below based on the number of acrylic substrates with a coat film to which cells had adhered. Regarding the evaluation of the biocompatibility, samples graded "C" or higher were regarded as acceptable. For practical use, samples graded "B" or higher are preferable. The results are shown in Table 1.

In addition, "-" shown in the column of Biocompatibility in Table 1 means that the sample could not be evaluated because the film was peeled off.

### (Evaluation standard)

"A":0
"B": Equal to or greater than 1 and equal to or smaller than 3
"C": Equal to or greater than 4 and equal to or smaller than 6
"D": Equal to or greater than 7

**[Table 1]**

| Table 1 | Makeup of curable composition | | | | | | | | Evaluation result | |
|---|---|---|---|---|---|---|---|---|---|---|
| | Composition No. | Component A | | Component B | | Component C | | Polymerization initiator | Substrate adhesion | Biocompatibility |
| | | Type | Note | Type | Note | Type | Note | | | |
| Example 1 | Curable composition 1 | ex (1) (48.5%) | Corresponding to Formula (1) | B (3) (48.5%) | Corresponding to Formula (A2) | - | - | Irg2959 (3%) | B | B |
| Example 2 | Curable composition 2 | ex (2) (48.5%) | Corresponding to Formula (1) | B (3) (48.5%) | Corresponding to Formula (A2) | - | - | Irg2959 (3%) | B | B |
| Example 3 | Curable composition 3 | ex (3) (48.5%) | Corresponding to Formula (2) | B (3) (48.5%) | Corresponding to Formula (A2) | - | - | Irg2959 (3%) | B | B |
| Example 4 | Curable composition 4 | ex (4) (48.5%) | Corresponding to Formula (2) | B (3) (48.5%) | Corresponding to Formula (A2) | - | - | Irg2959 (3%) | B | B |
| Example 5 | Curable composition 5 | ex (5) (48.5%) | Corresponding to Formula (3) | B (3) (48.5%) | Corresponding to Formula (A2) | - | - | Irg2959 (3%) | A | B |
| Example 6 | Curable composition 6 | ex (6) (48.5%) | Corresponding to Formula (3) | B (3) (48.5%) | Corresponding to Formula (A2) | - | - | Irg2959 (3%) | A | B |
| Example 7 | Curable composition 7 | ex (7) (48.5%) | Corresponding to Formula (3) | B (3) (48.5%) | Corresponding to Formula (A2) | - | - | Irg2959 (3%) | A | B |
| Example 8 | Curable composition 8 | ex (8) (48.5%) | Corresponding to Formula (3) | B (3) (48.5%) | Corresponding to Formula (A2) | - | - | Irg2959 (3%) | A | B |
| Example 9 | Curable composition 9 | ex (6) (48.5%) | Corresponding to Formula (3) | B (1) (48.5%) | Corresponding to Formula (A1) | - | - | Irg2959 (3%) | A | B |
| Example 10 | Curable composition 10 | ex (6) (48.5%) | Corresponding to Formula (3) | B (2) (48.5%) | Corresponding to Formula (A2) | - | - | Irg2959 (3%) | A | B |
| Example 11 | Curable composition 11 | ex (6) (48.5%) | Corresponding to Formula (3) | B (4) (48.5%) | Corresponding to Formula (A2) | - | - | Irg2959 (3%) | A | B |
| Example 12 | Curable composition 12 | ex (6) (4X.S%) | Corresponding to Formula (3) | B (5) (48.5%) | - | - | - | - | A | C |
| Example 13 | Curable composition 13 | ex (6) (67%) | Corresponding to Formula (3) | - | - | C (1) (30%) | R³¹ corresponds to Formula (I) | Irg2959 (3%) | B | A |
| Example 14 | Curable composition 14 | ex (6) (67%) | Corresponding to Formula (3) | - | - | C (2) (30%) | R³¹ corresponds to Formula (II) | Irg2959 (3%) | B | A |
| Example 15 | Curable composition 15 | ex (6) (67%) | Corresponding to Formula (3) | - | - | C (3) (30%) | R³¹ corresponds to Formula (III) | Irg2959 (3%) | B | B |
| Example 16 | Curable composition 16 | ex (6) (33.5%) | Corresponding to Formula (3) | B (3) (33.5%) | Corresponding to Formula (A2) | C (1) (30%) | R³¹ corresponds to Formula (I) | Irg2959 (3%) | A | A |
| Example 17 | Curable composition 17 | ex (6) (33.5%) | Corresponding to Formula (3) | B (3) (33.5%) | Corresponding to Formula (A2) | C (2) (30%) | R³¹ corresponds to Formula (II) | Irg2959 (3%) | A | A |
| Example 18 | Curable composition 18 | ex (7) (33.5%) | Corresponding to Formula (3) | B (1) (33.5%) | Corresponding to Formula (A1) | C (1) (30%) | R³¹ corresponds to Formula (I) | Irg2959 (3%) | A | A |
| Example 19 | Curable composition 19 | ex (7) (33.5%) | Corresponding to Formula (3) | B (1) (33.5%) | Corresponding to Formula (A1) | C (2) (30%) | R³¹ corresponds to Formula (II) | Irg2959 (3%) | A | A |
| Example 20 | Curable composition 20 | ex (7) (33.5%) | Corresponding to Formula (3) | B (4) (33.5%) | Corresponding to Formula (A2) | C (1) (30%) | R³¹ corresponds to Formula (I) | Irg2959 (3%) | A | A |
| Example 21 | Curable composition 21 | ex (7) (33.5%) | Corresponding to Formula (3) | B (4) (33.5%) | Corresponding to Formula (A2) | C (2) (30%) | R³¹ corresponds to Formula (II) | Irg2959 (3%) | A | A |
| Comparative Example 1 | Curable composition 22 | com (1) (48.5%) | - | B (1) (48.5%) | Corresponding to Formula (A1) | - | - | Irg2959 (3%) | C | D |
| Comparative Example 2 | Curable composition 23 | com (1) (48.5%) | - | B (2) (48.5%) | Corresponding to Formula (A2) | - | - | Irg2959 (3%) | C | D |
| Comparative Example 3 | Curable composition 24 | com (1) (48.5%) | - | B (3) (48.5%) | Corresponding to Formula (A2) | - | - | Irg2959 (3%) | C | C |
| Comparative Example 4 | Curable composition 25 | com (1) (48.5%) | - | B (4) (48.5%) | Corresponding to Formula (A2) | - | - | Irg2959 (3%) | C | D |
| Comparative Example 5 | Curable composition 26 | com (1) (67%) | - | - | - | C (1) (30%) | R³¹ corresponds to Formula (I) | Irg2959 (3%) | D | - |
| Comparative Example 6 | Curable composition 27 | com (1) (67%) | - | - | - | C (2) (30%) | R³¹ corresponds to Formula (II) | Irg2959 (3%) | D | - |
| Comparative Example 7 | Curable composition 28 | com (1) (67%) | - | - | - | C (3) (30%) | R³¹ corresponds to Formula (III) | Irg2959 (3%) | D | - |
| Comparative Example 8 | Curable composition 29 | - | - | B (1) (67%) | Corresponding to Formula (A1) | C (1) (30%) | R³¹ corresponds to Formula (I) | Irg2959 (3%) | D | - |
| Comparative Example 9 | Curable composition 30 | - | - | B (1) (67%) | Corresponding to Formula (A1) | C (2) (30%) | R³¹ corresponds to Formula (II) | Irg2959 (3%) | D | - |
| Comparative Example 10 | Curable composition 31 | - | - | B (1) (67%) | Corresponding to Formula (A1) | C (3) (30%) | R³¹ corresponds to Formula (III) | Irg2959 (3%) | D | - |
| Comparative Example 11 | Curable composition 32 | com (2) (67%) | - | - | - | C (1) (30%) | R³¹ corresponds to Formula (I) | Irg2959 (3%) | A | D |
| Comparative Example 12 | Curable composition 33 | com (2) (67%) | - | - | - | C (2) (30%) | R³¹ corresponds to Formula (II) | Irg2959 (3%) | A | D |
| Comparative Example 13 | Curable composition 34 | com (2) (67%) | - | - | - | C (3) (30%) | R³¹ corresponds to Formula (III) | Irg2959 (3%) | A | D |

From the results in Table 1, it was confirmed that according to the curable compositions of Examples, a cured product having excellent substrate adhesion and excellent biocompatibility was obtained.

By the comparison of Examples 1 to 8, it was confirmed that in a case where the compound represented by Formula (3) was used as the specific polyfunctional compound, the substrate adhesion was further improved.

By the comparison of Examples 9 to 12, it was confirmed that in a case where the compound represented by Formula (A1) or the compound represented by Formula (A2) was used as the specific polyfunctional (meth)acrylamide compound, the biocompatibility was further improved.

By the comparison of Examples 13 to 15, it was confirmed that in a case where the compound represented by Formula (C) was used as the betaine monomer, and R³¹ in the compound represented by Formula (C) is any of the group represented by Formula (I) or the group represented by formula (II), the biocompatibility was further improved.

Furthermore, by the comparison between Example 6 and Examples 16 and 17, it was confirmed that in a case where the curable composition contained all of the specific polyfunctional compound, the specific polyfunctional (meth)acrylamide compound, and the betaine monomer, both the high level of substrate adhesion and high level of biocompatibility could be accomplished.

On the other hand, it was confirmed that the cured product obtained from the curable composition of the comparative example did not satisfy the desired requirements.

## Claims

1. A curable composition comprising:
one or more kinds of polyfunctional compounds selected from the group consisting of a compound represented by Formula (1), a compound represented by Formula (2), and a compound represented by Formula (3); and
one or more kinds of compounds selected from the group consisting of a compound represented by Formula (A) and a betaine monomer, 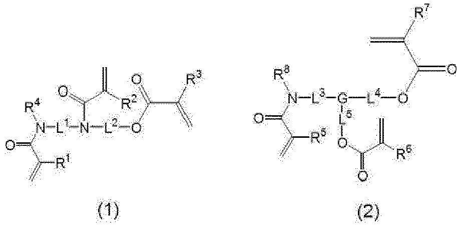 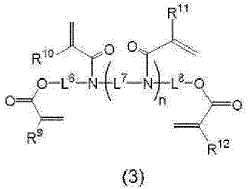
in Formula (1), R¹ to R⁴ each independently represent a hydrogen atom or an alkyl group, L¹ and L² each independently represent an alkylene group which may contain one or more kinds of divalent linking groups selected from the group consisting of -O-, -S-, -NR^{A}-, and -CO-, R^{A} represents a hydrogen atom or an alkyl group,
in Formula (2), R⁵ to R⁸ each independently represent a hydrogen atom or an alkyl group, L³ represents a single bond or an alkylene group which may contain one or more kinds of divalent linking groups selected from the group consisting of -O-, -S-, -NR^{A}- and -CO-, L⁴ and L⁵ each independently represent an alkylene group which may contain one or more kinds of divalent linking groups selected from the group consisting of -O-, -S-, -NR^{A}-, and -CO-, R^{A} represents a hydrogen atom or an alkyl group, G represents a trivalent linking group,
in Formula (3), R⁹ to R¹² each independently represent a hydrogen atom or an alkyl group, L⁶ to L⁸ each independently represent an alkylene group which may contain one or more kinds of divalent linking groups selected from the group consisting of -O-, -S-, -NR^{A}-, and -CO-, R^{A} represents a hydrogen atom or an alkyl group, n represents an integer of 0 to 3, L⁷s may be the same as or different from each other in a case where there is a plurality of L⁷s, R¹¹s may be the same as or different from each other in a case where there is a plurality of R¹¹s, 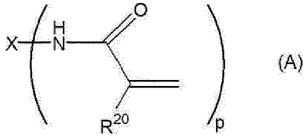
in Formula (A), R²⁰ represents a hydrogen atom or a methyl group, X represents a p-valent linking group, p represents an integer of 2 to 4, and a plurality of R²⁰s may be the same as or different from each other.

2. The curable composition according to claim 1, comprising:
the polyfunctional compound selected from the group consisting of the compound represented by Formula (1), the compound represented by Formula (2), and the compound represented by Formula (3);
the compound represented by Formula (A); and
the betaine monomer.

3. The curable composition according to claim 1 or 2,
wherein the polyfunctional compound is the compound represented by Formula (3).

4. The curable composition according to any one of claims 1 to 3,
wherein the compound represented by Formula (A) is a compound represented by Formula (A1) or a compound represented by Formula (A2), 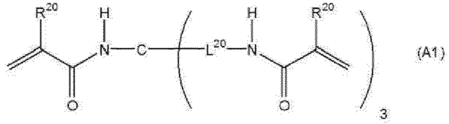 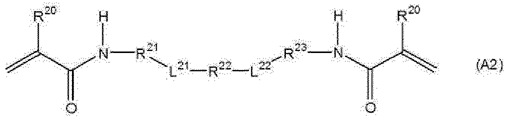 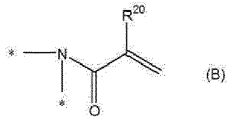
in Formula (A1), R²⁰ each independently represents a hydrogen atom or a methyl group, L²⁰ each independently represents -O-, an alkylene group having 2 to 4 carbon atoms, or a divalent linking group obtained by combining these, a plurality of R²⁰s may be the same as or different from each other, a plurality of L²⁰s may be the same as or different from each other,
in Formula (A2), R²⁰ each independently represents a hydrogen atom or a methyl group, R²¹ and R²³ each independently represent -O-, an alkylene group having 1 to 4 carbon atoms, or a divalent linking group obtained by combining these, R²² represents -O-, an alkylene group having 1 to 4 carbon atoms, a group represented by Formula (B), or a divalent linking group obtained by combining these, L²¹ and L²² each independently represent a single bond or a group represented by Formula (B), a plurality of R²⁰s may be the same as or different from each other,
in Formula (B), R²⁰ represents a hydrogen atom or a methyl group, and * represents a binding position.

5. The curable composition according to any one of claims 1 to 4,
wherein the betaine monomer is a compound represented by Formula (C), 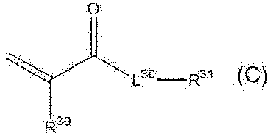
in Formula (C), R³⁰ represents a hydrogen atom or an alkyl group, L³⁰ represents -O-or -NR^{A}-, R³¹ represents a monovalent group represented by Formula (I), a monovalent group represented by Formula (II), or a monovalent group represented by Formula (III), R^{A} represents a hydrogen atom or an alkyl group, 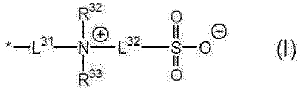 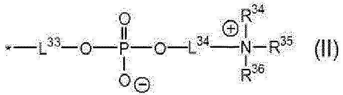 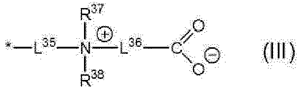
in Formula (I), L³¹ and L³² each independently represent a divalent linking group, R³² and R³³ each independently represent an alkyl group, * represents a binding position,
in Formula (II), L³³ and L³⁴ each independently represent a divalent linking group, R³⁴ to R³⁶ each independently represent an alkyl group, * represents a binding position,
in Formula (III), L³⁵ and L³⁶ each independently represent a divalent linking group, R³⁷ and R³⁸ each independently represent an alkyl group, and * represents a binding position.

6. The curable composition according to claim 5,
wherein R³¹ represents the monovalent group represented by Formula (I) or the monovalent group represented by Formula (II).

7. A film comprising:
a polymer compound containing one or more kinds of repeating units selected from the group consisting of a repeating unit derived from a compound represented by Formula (1), a repeating unit derived from a compound represented by Formula (2), and a repeating unit derived from a compound represented by Formula (3); and
one or more kinds of repeating units selected from the group consisting of a repeating unit derived from a compound represented by Formula (A) and a repeating unit derived from a betaine monomer, 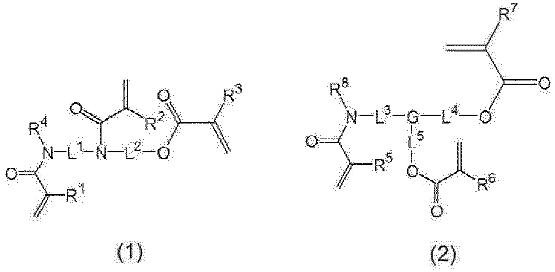 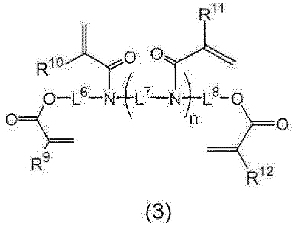
in Formula (1), R¹ to R⁴ each independently represent a hydrogen atom or an alkyl group, L¹ and L² each independently represent an alkylene group which may contain one or more kinds of divalent linking groups selected from the group consisting of -O-, -S-, -NR^{A}-, and -CO-, R^{A} represents a hydrogen atom or an alkyl group,
in Formula (2), R⁵ to R⁸ each independently represent a hydrogen atom or an alkyl group, L³ represents a single bond or an alkylene group which may contain one or more kinds of divalent linking groups selected from the group consisting of -O-, -S-, -NR^{A}- and -CO-, L⁴ and L⁵ each independently represent an alkylene group which may contain one or more kinds of divalent linking groups selected from the group consisting of -O-, -S-, -NR^{A}-, and -CO-, R^{A} represents a hydrogen atom or an alkyl group, G represents a trivalent linking group,
in Formula (3), R⁹ to R¹² each independently represent a hydrogen atom or an alkyl group, L⁶ to L⁸ each independently represent an alkylene group which may contain one or more kinds of divalent linking groups selected from the group consisting of -O-, -S-, -NR^{A}-, and -CO-, R^{A} represents a hydrogen atom or an alkyl group, n represents an integer of 0 to 3, L⁷s may be the same as or different from each other in a case where there is a plurality of L⁷s, R¹¹s may be the same as or different from each other in a case where there is a plurality of R¹¹s, 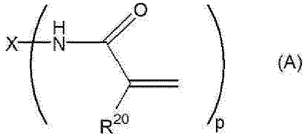
in Formula (A), R²⁰ represents a hydrogen atom or a methyl group, X represents a p-valent linking group, p represents an integer of 2 to 4, and a plurality of R²⁰s may be the same as or different from each other.

8. A cured product formed by curing the curable composition according to any one of claims 1 to 6.

9. The cured product according to claim 8 that is in the form of a film.

10. The cured product according to claim 8 or 9 that is used as a biomaterial.

11. A medical member comprising:
a substrate; and
the cured product according to any one of claims 8 to 10 that is disposed on the substrate.

## Patentansprüche

1. Härtbare Zusammensetzung, enthaltend:
eine oder mehrere Arten von polyfunktionellen Verbindungen, ausgewählt aus der Gruppe, bestehend aus einer Verbindung mit der Formel (1), einer Verbindung mit der Formel (2) und einer Verbindung mit der Formel (3), und
eine oder mehrere Arten von Verbindungen, ausgewählt aus der Gruppe, bestehend aus einer Verbindung mit der Formel (A) und einem Betain-Monomer 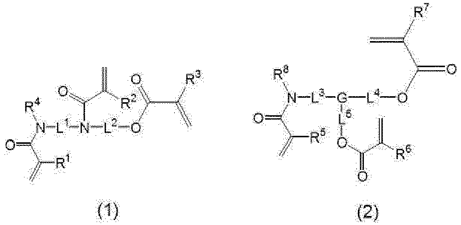 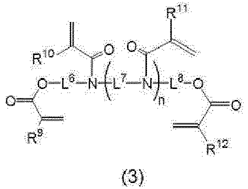
worin in der Formel (1) R¹ bis R⁴ jeweils unabhängig ein Wasserstoffatom oder eine Alkylgruppe sind, L¹ und L² jeweils unabhängig eine Alkylengruppe sind, die eine oder mehrere Arten von bivalenten Bindegruppen enthalten können, ausgewählt aus der Gruppe, bestehend aus -O-, -S-, -NR^{A}- und -CO-, worin R^{A} ein Wasserstoffatom oder eine Alkylgruppe ist,
worin in der Formel (2) R⁵ bis R⁸ jeweils unabhängig ein Wasserstoffatom oder eine Alkylgruppe sind, L³ eine Einfachbindung oder eine Alkylengruppe ist, die eine oder mehrere Arten von bivalenten Bindegruppen enthalten kann, ausgewählt aus der Gruppe, bestehend aus -O-, -S-, -NR^{A}- und -CO-, L⁴ und L⁵ jeweils unabhängig eine Alkylengruppe sind, die eine oder mehrere Arten von bivalenten Bindegruppen enthalten kann, ausgewählt aus der Gruppe, bestehend aus -O-, -S-, -NR^{A}- und -CO-, R^{A} ein Wasserstoffatom oder eine Alkylgruppe ist, G eine trivalente Bindegruppe ist,
worin in der Formel (3) R⁹ bis R¹² jeweils unabhängig ein Wasserstoffatom oder eine Alkylgruppe sind, L⁶ bis L⁸ jeweils unabhängig eine Alkylengruppe sind, die eine oder mehrere Arten von bivalenten Bindegruppen enthalten kann, ausgewählt aus der Gruppe, bestehend aus -O-, -S-, -NR^{A}- und -CO-, R^{A} ein Wasserstoffatom oder eine Alkylgruppe ist, n eine ganze Zahl von 0 bis 3 ist, die Gruppen L⁷ gleich oder verschieden voneinander sein können, wenn es mehrere L⁷ gibt, die Gruppen R¹¹ gleich oder verschieden voneinander sein können, wenn es mehrere R¹¹ gibt, 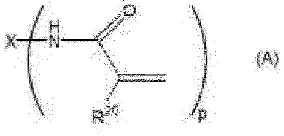
worin in der Formel (A) R²⁰ ein Wasserstoffatom oder eine Methylgruppe ist, X eine p-valente Bindegruppe ist, p eine ganze Zahl von 2 bis 4 ist und eine Vielzahl von R²⁰ gleich oder verschieden voneinander sein kann.

2. Härtbare Zusammensetzung gemäß Anspruch 1, enthaltend:
die polyfunktionelle Verbindung, ausgewählt aus der Gruppe, bestehend aus der Verbindung mit der Formel (1), der Verbindung mit der Formel (2) und der Verbindung mit der Formel (3),
die Verbindung mit der Formel (A) und
das Betain-Monomer.

3. Härtbare Zusammensetzung gemäß Anspruch 1 oder 2, worin die polyfunktionelle Verbindung die Verbindung mit der Formel (3) ist.

4. Härtbare Zusammensetzung gemäß einem der Ansprüche 1 bis 3, worin die Verbindung mit der Formel (A) eine Verbindung mit der Formel (A1) oder eine Verbindung mit der Formel (A2) ist, 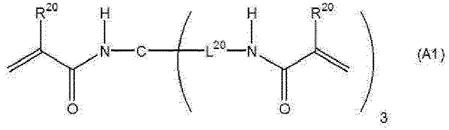 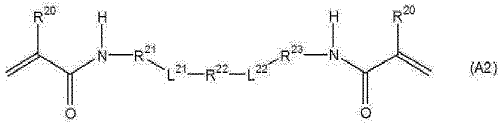 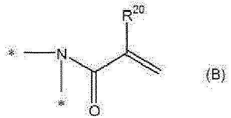
worin in der Formel (A1) R²⁰ jeweils unabhängig ein Wasserstoffatom oder eine Methylgruppe ist, L²⁰ unabhängig -O-, eine Alkylengruppe mit 2 bis 4 Kohlenstoffatomen oder eine bivalente Bindegruppe ist, erhalten durch Kombinieren dieser, eine Vielzahl von R²⁰ gleich oder verschieden voneinander sein kann, eine Vielzahl von L²⁰ gleich oder verschieden voneinander sein kann,
worin in der Formel (A2) R²⁰ jeweils unabhängig ein Wasserstoffatom oder eine Methylgruppe ist, R²¹ und R²³ jeweils unabhängig -O-, eine Alkylengruppe mit 1 bis 4 Kohlenstoffatomen oder eine bivalente Bindegruppe sind, erhalten durch Kombinieren dieser, R²² -O-, eine Alkylengruppe mit 1 bis 4 Kohlenstoffatomen, eine Gruppe mit der Formel (B) oder eine bivalente Bindegruppe ist, erhalten durch Kombinieren dieser, L²¹ und L²² jeweils unabhängig eine Einfachbindung oder eine Gruppe mit der Formel (B) sind, wobei eine Vielzahl von R²⁰ gleich oder verschieden voneinander sein kann,
worin in der Formel (B) R²⁰ ein Wasserstoffatom oder eine Methylgruppe ist und * eine Bindeposition ist.

5. Härtbare Zusammensetzung gemäß einem der Ansprüche 1 bis 4, worin das Betain-Monomer eine Verbindung mit der Formel (C) ist, 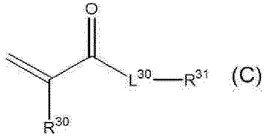
worin in der Formel (C) R³⁰ ein Wasserstoffatom oder eine Alkylgruppe ist, L³⁰ -O- oder -NR^{A}- ist, worin R³¹ eine monovalente Gruppe mit der Formel (I), eine monovalente Gruppe mit der Formel (II) oder eine monovalente Gruppe mit der Formel (III) ist, R^{A} ein Wasserstoffatom oder eine Alkylgruppe ist, 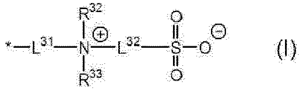 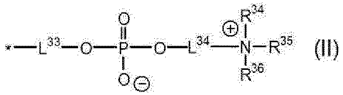 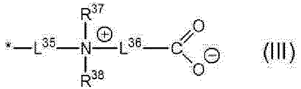
worin in der Formel (I) L³¹ und L³² jeweils unabhängig eine bivalente Bindegruppe sind, R³² und R³³ jeweils unabhängig eine Alkylgruppe sind, * eine Bindeposition ist,
worin in der Formel (II) L³³ und L³⁴ jeweils unabhängig eine bivalente Bindegruppe sind, R³⁴ bis R³⁶ jeweils unabhängig eine Alkylgruppe sind, * eine Bindeposition ist,
worin in der Formel (III) L³⁵ und L³⁶ jeweils unabhängig eine bivalente Bindegruppe sind, R³⁷ bis R³⁸ jeweils unabhängig eine Alkylgruppe sind, und * eine Bindeposition ist.

6. Härtbare Zusammensetzung gemäß Anspruch 5, worin R³¹ die monovalente Gruppe mit der Formel (I) oder die monovalente Gruppe mit der Formel (II) ist.

7. Film, enthaltend:
eine Polymerverbindung, die eine oder mehrere Arten von Wiederholungseinheiten enthält, ausgewählt aus der Gruppe, bestehend aus einer Wiederholungseinheit, die von einer Verbindung mit der Formel (I) stammt, einer Wiederholungseinheit, die von einer Verbindung mit der Formel (II) stammt, und einer Wiederholungseinheit, die von einer Verbindung mit der Formel (III) stammt, und eine oder mehrere Arten von Wiederholungseinheiten,
ausgewählt aus der Gruppe, bestehend aus einer Wiederholungseinheit, die von einer Verbindung mit der Formel (A) stammt, und einer Wiederholungseinheit, die von einem Betain-Monomer stammt, 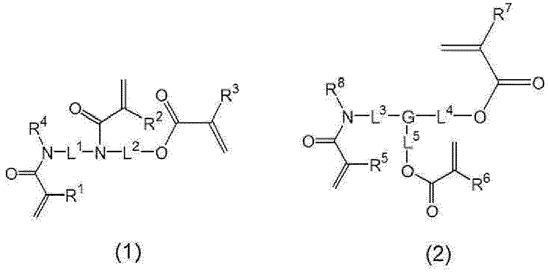 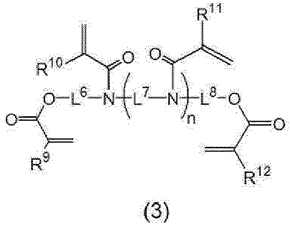
worin in der Formel (1) R¹ bis R⁴ jeweils unabhängig ein Wasserstoffatom oder eine Alkylgruppe sind, L¹ und L² jeweils unabhängig eine Alkylengruppe sind, die eine oder mehrere Arten von bivalenten Bindegruppen enthalten kann**,** ausgewählt aus der Gruppe, bestehend aus -O-, -S-, -NR^{A}- und -CO-, worin R^{A} ein Wasserstoffatom oder eine Alkylgruppe ist,
worin in der Formel (2) R⁵ bis R⁸ jeweils unabhängig ein Wasserstoffatom oder eine Alkylgruppe sind, L³ eine Einfachbindung oder eine Alkylengruppe ist, die eine oder mehrere Arten von bivalenten Bindegruppen enthalten kann, ausgewählt aus der Gruppe, bestehend aus -O-, -S-, -NR^{A}- und -CO-, L⁴ und L⁵ jeweils unabhängig eine Alkylengruppe sind, die eine oder mehrere Arten von bivalenten Bindegruppen enthalten kann, ausgewählt aus der Gruppe, bestehend aus -O-, -S-, -NR^{A}- und -CO-, R^{A} ein Wasserstoffatom oder eine Alkylgruppe ist, G eine trivalente Bindegruppe ist,
worin in der Formel (3) R⁹ bis R¹² jeweils unabhängig ein Wasserstoffatom oder eine Alkylgruppe sind, L⁶ bis L⁸ jeweils unabhängig eine Alkylengruppe sind, die eine oder mehrere Arten von bivalenten Bindegruppen enthalten können, ausgewählt aus der Gruppe, bestehend aus -O-, -S-, -NR^{A}- und -CO-, R^{A} ein Wasserstoffatom oder eine Alkylgruppe ist, n eine ganze Zahl von 0 bis 3 ist, die Gruppen L⁷ gleich oder verschieden voneinander sein können, wenn es mehrere L⁷ gibt, die Gruppen R¹¹ gleich oder verschieden voneinander sein können, wenn es mehrere R¹¹ gibt, 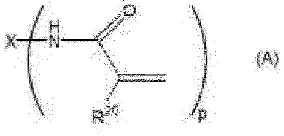
worin in der Formel (A) R²⁰ ein Wasserstoffatom oder eine Methylgruppe ist, X eine p-valente Bindegruppe ist, p eine ganze Zahl von 2 bis 4 ist und eine Vielzahl von R²⁰ gleich oder verschieden voneinander sein kann.

8. Gehärtetes Produkt, gebildet durch Härten der härtbaren Zusammensetzung gemäß einem der Ansprüche 1 bis 6.

9. Gehärtetes Produkt gemäß Anspruch 8, das in der Form eines Films vorliegt.

10. Gehärtetes Produkt gemäß Anspruch 8 oder 9, das als Biomaterial verwendet wird.

11. Medizinisches Teil, enthaltend:
ein Substrat und
das gehärtete Produkt gemäß einem der Ansprüche 8 bis 10, das auf dem Substrat angeordnet ist.

## Revendications

1. Composition durcissable comprenant :
un ou plusieurs types de composés polyfonctionnels sélectionnés à partir du groupe consistant en un composé représenté par la Formule (1), un composé représenté par la Formule (2) et un composé représenté par la Formule (3) ; et
un ou plusieurs types de composés sélectionnés à partir du groupe consistant en un composé représenté par la Formule (A) et d'un monomère de bétaïne, 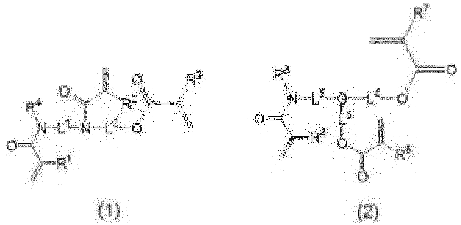 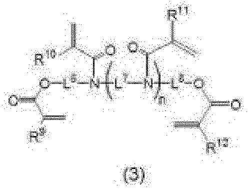
dans la Formule (1), R¹ à R⁴ représentent chacun indépendamment un atome d'hydrogène ou un groupe alkyle, L¹ et L² représentent chacun indépendamment un groupe alkylène qui peut contenir un ou plusieurs types de groupes de liaison divalents sélectionnés à partir du groupe consistant en -O-, -S-, -NR^{A}- et -CO-, R^{A} représente un atome d'hydrogène ou un groupe alkyle,
dans la Formule (2), R⁵ à R⁸ représentent chacun indépendamment un atome d'hydrogène ou un groupe alkyle, L³ représente une liaison simple ou un groupe alkylène qui peut contenir un ou plusieurs types de groupes de liaison divalents sélectionnés à partir du groupe consistant en -O-, -S-, -NR^{A}- et -CO-, L⁴ et L⁵ représentent chacun indépendamment un groupe alkylène qui peut contenir un ou plusieurs types de groupes de liaison divalents sélectionnés à partir du groupe consistant en -O-, -S-, -NR^{A}-, et -CO-, R^{A} représente un atome d'hydrogène ou un groupe alkyle, G représente un groupe de liaison trivalent,
dans la Formule (3), R⁹ à R¹² représentent chacun indépendamment un atome d'hydrogène ou un groupe alkyle, L⁶ à L⁸ représentent chacun indépendamment un groupe alkylène qui peut contenir un ou plusieurs types de groupes de liaison divalents sélectionnés à partir du groupe consistant en -O-, -S-, -NR^{A}- et -CO-, R^{A} représente un atome d'hydrogène ou un groupe alkyle, n représente un nombre entier de 0 à 3, les L⁷ peuvent être identiques ou différents les uns des autres dans un cas où il y a un pluralité de L⁷, les R¹¹ peuvent être identiques ou différent les uns des autres dans un cas où il y a une pluralité de R¹¹, 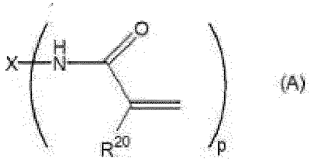
dans la Formule (A), R²⁰ représente un atome d'hydrogène ou un groupe méthyle, X représente un groupe de liaison p-valent, p représente un nombre entier de 2 à 4, et une pluralité de R²⁰ peuvent être identiques ou différents les uns des autres.

2. Composition durcissable selon la revendication 1, comprenant :
le composé polyfonctionnel sélectionné à partir du groupe consistant en le composé représenté par la Formule (1), le composé représenté par la Formule (2) et le composé représenté par la Formule (3) ;
le composé représenté par la Formule (A) ; et
le monomère de bétaïne.

3. Composition durcissable selon la revendication 1 ou 2,
dans laquelle le composé polyfonctionnel est le composé représenté par la Formule (3).

4. Composition durcissable selon l'une quelconque des revendications 1 à 3,
dans laquelle le composé représenté par la Formule (A) est un composé représenté par la Formule (A1) ou un composé représenté par la Formule (A2), 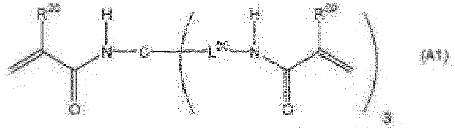 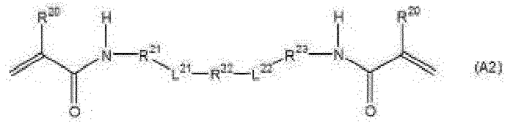 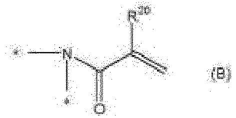
dans la Formule (A1), R²⁰ représente chacun indépendamment un atome d'hydrogène ou un groupe méthyle, L²⁰ représente chacun indépendamment -O-, un groupe alkylène présentant 2 à 4 atomes de carbone, ou un groupe de liaison divalent obtenu en combinant ceux-ci, une pluralité de R²⁰ peuvent être identiques ou différents les uns des autres, une pluralité de L²⁰ peuvent être identiques ou différents les uns des autres,
dans la Formule (A2), R²⁰ représente chacun indépendamment un atome d'hydrogène ou un groupe méthyle, R²¹ et R²³ représentent chacun indépendamment -O-, un groupe alkylène présentant 1 à 4 atomes de carbone, ou un groupe de liaison divalent obtenu en combinant ceux-ci, R²² représente -O-, un groupe alkylène présentant 1 à 4 atomes de carbone, un groupe représenté par la Formule (B), ou un groupe de liaison divalent obtenu en combinant ceux-ci, L²¹ et L²² représentent chacun indépendamment une liaison simple ou un groupe représenté par la Formule (B), une pluralité de R²⁰ peuvent être identiques ou différents les uns des autres,
dans la Formule (B), R²⁰ représente un atome d'hydrogène ou un groupe méthyle, et * représente une position de liaison.

5. Composition durcissable selon l'une quelconque des revendications 1 à 4,
dans laquelle le monomère de bétaïne est un composé représenté par la Formule (C), 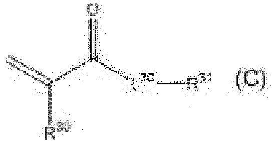
dans la Formule (C), R³⁰ représente un atome d'hydrogène ou un groupe alkyle, L³⁰ représente -O- ou -NR^{A}-, R³¹ représente un groupe monovalent représenté par la Formule (I), un groupe monovalent représenté par la Formule (II), ou un groupe monovalent représenté par la Formule (III), RA représente un atome d'hydrogène ou un groupe alkyle, 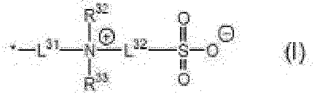 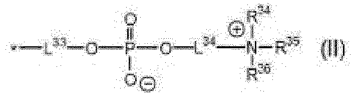 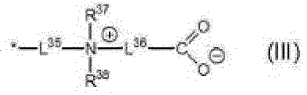
dans la Formule (I), L³¹ et L³² représentent chacun indépendamment un groupe de liaison divalent, R³² et R³³ représentent chacun indépendamment un groupe alkyle, * représente une position de liaison,
dans la Formule (II), L³³ et L³⁴ représentent chacun indépendamment un groupe de liaison divalent, R³⁴ à R³⁶ représentent chacun indépendamment un groupe alkyle, * représente une position de liaison,
dans la Formule (III), L³⁵ et L³⁶ représentent chacun indépendamment un groupe de liaison divalent, R³⁷ et R³⁸ représentent chacun indépendamment un groupe alkyle et * représente une position de liaison.

6. Composition durcissable selon la revendication 5,
dans laquelle R³¹ représente le groupe monovalent représenté par la Formule (I) ou le groupe monovalent représenté par la Formule (II).

7. Film comprenant :
un composé polymère contenant un ou plusieurs types d'unités répétitives sélectionnés à partir du groupe consistant en une unité répétitive dérivée d'un composé représenté par la Formule (1), une unité répétitive dérivée d'un composé représenté par la Formule (2), et une unité répétitive dérivée à partir d'un composé représenté par la Formule (3) ; et
un ou plusieurs types d'unités répétitives sélectionnés à partir du groupe consistant en une unité répétitive dérivée d'un composé représenté par la Formule (A) et une unité répétitive dérivée d'un monomère de bétaïne, 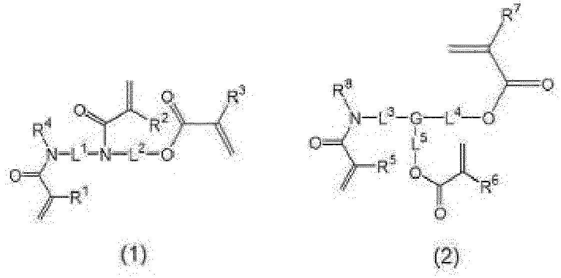 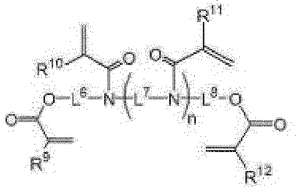
dans la Formule (1), R¹ à R⁴ représentent chacun indépendamment un atome d'hydrogène ou un groupe alkyle, L¹ et L² représentent chacun indépendamment un groupe alkylène qui peut contenir un ou plusieurs types de groupes de liaison divalents sélectionnés à partir du groupe consistant en -O-, -S-, -NR^{A}- et -CO-, R^{A} représente un atome d'hydrogène ou un groupe alkyle,
dans la Formule (2), R⁵ à R⁸ représentent chacun indépendamment un atome d'hydrogène ou un groupe alkyle, L³ représente une liaison simple ou un groupe alkylène qui peut contenir un ou plusieurs types de groupes de liaison divalents sélectionnés à partir du groupe consistant en -O-, -S-, -NR^{A}- et -CO-, L⁴ et L⁵ représentent chacun indépendamment un groupe alkylène qui peut contenir un ou plusieurs types de groupes de liaison divalents sélectionnés à partir du groupe consistant en -O-, -S-, -NR^{A}-, et -CO-, R^{A} représente un atome d'hydrogène ou un groupe alkyle, G représente un groupe de liaison trivalent,
dans la Formule (3), R⁹ à R¹² représentent chacun indépendamment un atome d'hydrogène ou un groupe alkyle, L⁶ à L⁸ représentent chacun indépendamment un groupe alkylène qui peut contenir un ou plusieurs types de groupes de liaison divalents sélectionnés à partir du groupe consistant en -O-, -S-, -NR^{A}- et -CO-, R^{A} représente un atome d'hydrogène ou un groupe alkyle, n représente un nombre entier de 0 à 3, les L⁷ peuvent être identiques ou différents les uns des autres dans un cas où il y a un pluralité de L⁷, les R¹¹ peuvent être identiques ou différent les uns des autres dans un cas où il y a une pluralité de R¹¹, 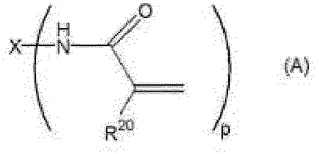
dans la Formule (A), R²⁰ représente un atome d'hydrogène ou un groupe méthyle, X représente un groupe de liaison p-valent, p représente un nombre entier de 2 à 4, et une pluralité de R²⁰ peuvent être identiques ou différents les uns des autres.

8. Produit durci formé par durcissement de la composition durcissable selon l'une quelconque des revendications 1 à 6.

9. Produit durci selon la revendication 8 qui se présente sous la forme d'un film.

10. Produit durci selon la revendication 8 ou 9 qui est utilisé en tant que biomatériau.

11. Membre médical comprenant :
un substrat ; et le produit durci selon l'une quelconque des revendications 8 à 10 qui est disposé sur le substrat.
